# EUROPEAN PATENT APPLICATION

(11) **EP 3 984 546 A2**
(43) Date of publication of application: **20.04.2022**
(21) Application number: 20810087.5
(22) Date of filing: 15.05.2020
(51) Int. Cl.: A61K 38/00, A61K 31/713, A61K 38/16, C12N 15/63, C12N 15/85, C07K 14/245, A61P 35/00

(54) **GENE THERAPY WITH THE GENES HOKD AND LDRB FOR CANCER TREATMENTS**

(30) Priority: 15.05.2019 ES 201930428; 15.05.2019 ES 201930427
(71) Applicant: Universidad de Granada, 18071 Granada (ES); Servicio Andaluz de Salud, 41071 Sevilla (ES)
(72) Inventor: BOULAIZ TASSI, Houria, 18071 Granada (ES); MARCHAL CORRALES, Juan Antonio, 18071 Granada (ES); GRIÑÁN LISÓN, Carmen, 18071 Granada (ES); JIMÉNEZ GONZÁLEZ, Gema, 18071 Granada (ES); JIMÉNEZ MARTÍNEZ, Yaiza, 18012 Granada (ES)
(74) Representative: Hoffmann Eitle
(86) International application number: PCT/ES2020/070315
(87) International publication number: WO 2020/234498

(57) **Abstract**

The invention relates to the isolated RNA or DNA polynucleotides *hokD* and *IdrB* for use in medicine, particularly in gene therapy for cancer treatment. Also described are compositions comprising such polynucleotides or related genetic structures, and a method for treating proliferative disorders, preferably cancer.

## Description

### FIELD OF THE ART

The present invention is comprised in the field of gene therapy. More specifically, the present invention relates to toxins, *hokD* and *IdrB,* with an application in the field of gene therapy and particularly for cancer treatment.

### STATE OF THE ART

Cancer is a disease that has an enormous impact on the entire world. In the United States, 1,685,210 new cases of cancer are expected to be diagnosed for 2016 and 595,690 people are expected to die from this cause. The cancer mortality rate has dropped by 23% since 1991, which translates into more than 1.7 million deaths prevented up to 2012 [1]. This is due to the improved diagnosis and intervention; however, conventional treatments (chemotherapy, radiotherapy, surgery, and hormone therapy) have certain limitations. Furthermore, patients who are subjected to current systemic therapies will experience a number of side effects ranging from nausea to infertility [2]. This shows that it is necessary to reduce the chemotherapy or radiation dose to below the most effective levels, or to entirely withdraw a particular treatment. Therefore, research in new alternative and more efficient therapies is required.

Technologies for transferring genes into tumor cells are providing new possibilities for cancer therapy [3]. One potential approach involves the genetic modification of tumor cells based on the transfer of suicide genes [4]. Suicide gene therapy is based on the delivery to tumor cells of a gene encoding a cytotoxic protein. This therapy can be divided into two categories: indirect gene therapy using an enzyme-activating prodrug, which allows the conversion of a prodrug into a lethal drug in tumor cells; and direct gene therapy using a toxin gene, which incorporates genes expressing toxic molecules which may affect cell membrane stability and reduce tumor cell viability [5,6].

More strategies being developed involve indirect genetic therapy using an enzyme-activating prodrug [7]. One of the most widely studied therapeutic strategies is based on transfection of the herpes simplex virus thymidine kinase (HSV-TK) gene with the administration of ganciclovir (GCV). HSV-TK converts the antiviral drug GCV into a monophosphorylated molecule which is then metabolized into the toxic form of triphosphate by cellular kinases, inhibiting DNA synthesis and causing cell death [8,9]. Another system is a cytosine deaminase (CD) (an enzyme found in bacteria and fungi, but absent in mammalian cells) with the administration of 5-fluorocytosine (5-FC). This system is based on the capacity of CD to convert non-toxic 5-FC into 5-fluorouracil (5-FU), a potent cytotoxic chemotherapeutic, which is later transformed by means of cell enzymes into potent pyrimidine antimetabolites and causes the inhibition of cell proliferation and cell death [10,11]. One alternative in suicide gene therapy for cancer is use of the toxin gene [12]. Unlike conventional suicide gene therapy, uses of the toxin gene have more advantages. The presence of a prodrug is not required for the system to be effective. Furthermore, the toxicity of the metabolites is reduced and has not limited bioavailability due to the lack of prodrugs. Some bacterial toxins have been studied for use in gene therapy for cancer [13,14].

Almost all bacteria contain several toxin-antitoxin (TA) modules. The toxin products of these modules target various cell functions to regulate cell growth and death. These toxins are generally co-expressed with their associated antitoxins in operons called toxin-antitoxin (TA) modules [15]. One of the genes of the operon encodes an unstable antitoxin and another gene encodes a stable toxin, where the overlap of both loci is common. Under normal conditions of growth, the modules form stable complexes. However, stress-induced proteases preferably eliminate the unstable antitoxins, releasing free toxins to inhibit various cell functions [16,17].

These TA systems are classified into three groups (type I, II, III) based on the function of the antitoxin [18]. The *Escherichia coli* K-12 genome encodes at least 36 assumed TA systems which consist of type I and type II systems. In type I TA systems, the expression of the toxin gene is regulated by an antisense RNA transcribed from the toxin module but in the reverse orientation. This antisense RNA impedes the translation of the mRNA toxin [19,20].

The *E. coli* K-12 chromosome contains different type I TA systems, and one of these systems is the hok/sok family containing six homologous loci (A, B, C, D, E, and X) [21]. The gene *hokD* encodes a highly toxic 52-amino acid transmembrane protein which irreversibly damages the cell membrane and, therefore, is lethal for the host cell. Effects of the toxin result in the loss of membrane potential, arrest of respiration, death of the host cell, and change in cell morphology [22,23]. Another family of the type I TA system also encoded by *E. coli* K-12 is the family of the gene Idr/rdl.

Due to the high prevalence of cancer in recent years, the development of new and more effective therapies that cause fewer side effects is needed. The development of gene therapy for cancer based on the use of suicide genes that can damage the tumor cell and do not require a prodrug due to its lethal effect is one of the objectives of the new gene therapy strategies.

The applicants do not know of any disclosure in the prior art relating to the use of the genes *hokD* and *IdrB* in gene therapy. Particularly, there are no earlier disclosures about their use in gene therapy for cancer.

### DESCRIPTION OF THE FIGURES

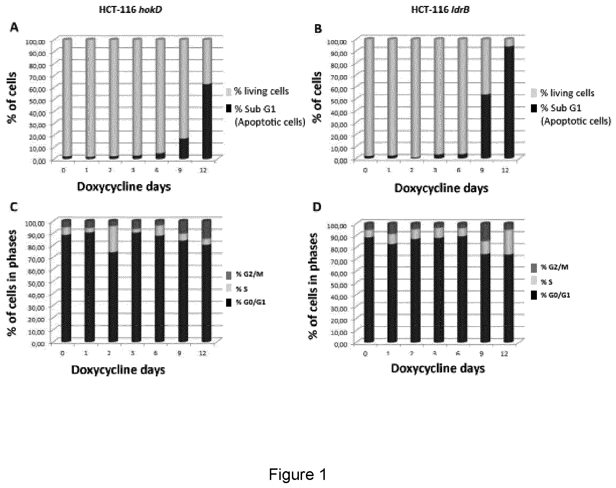

**Figure 1****.** Apoptosis (SubG1) and analysis of the cell cycle (G0/G1, S, G2/M) of HCT-116 *hokD* and HCT-116 *IdrB* cells at different time points (0, 1, 2, 3, 6, 9, and 12 days) of gene induction by Dox. The genes *hokD* (A) and *IdrB* (B) increase the percentage of apoptotic cells from day 6 of gene expression. (C) Histogram of the HCT-116 *hokD* cell cycle for different days of induction with Dox; the graph shows the increase in the percentage of cells in the S phase by more than three-fold in comparison with the control. At 9 and 12 days after gene expression, the percentage of cells in G2/M is, respectively, two and three fold higher than the control. (D) Histogram of the HCT-116 *IdrB* cell cycle for different days of induction with Dox; the graph shows twice the percentage of cells in G2/M after 9 and 12 days of gene expression in comparison with the control.
**Figure 2****.** Analysis of the cell cycle (G0/G1, S, G2/M, and Sub-G1). The representative histograms indicate the number of cells (count; vertical axis) against the DNA content by means of staining with PI (PI; horizontal axis) of HCT-116 *hokD* (A) and HCT-116 *IdrB* (B) at different time periods (0, 1, 2, 3, 6, 9, and 12 days) of induction of the gene Dox.

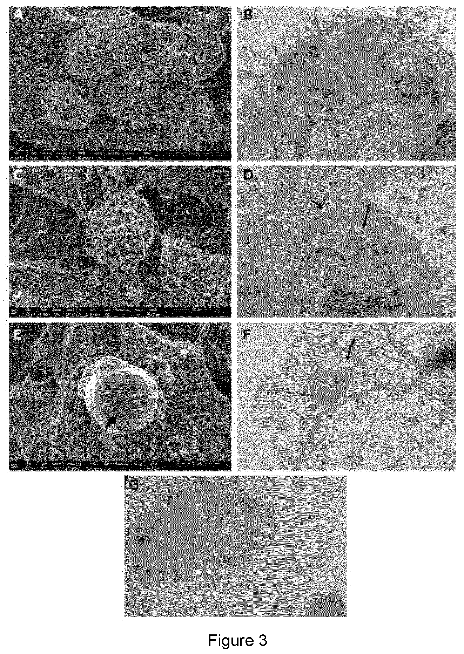

**Figure 3****.** (A, C, E) Scanning electron microscopy (SEM) and (B, D, F, G) transmission electron microscopy analysis of colon cancer HCT-116 cell line. Microphotographs (A) and (B) represent HCT-116 control cells; (C, D, E, F, G) represent HCT116 *hokD* after 3 days of gene induction. In comparison with the control, (C) apoptotic cells covered by a number of apoptotic bodies and (E) some cells with pores and "flat surfaces" (black arrow) are observed. At the ultra-structural level, (D, F) HCT-116 *hokD* cells show dilated mitochondria (black arrows) and (G) disintegrated cells. Scale bar = 5 µm.

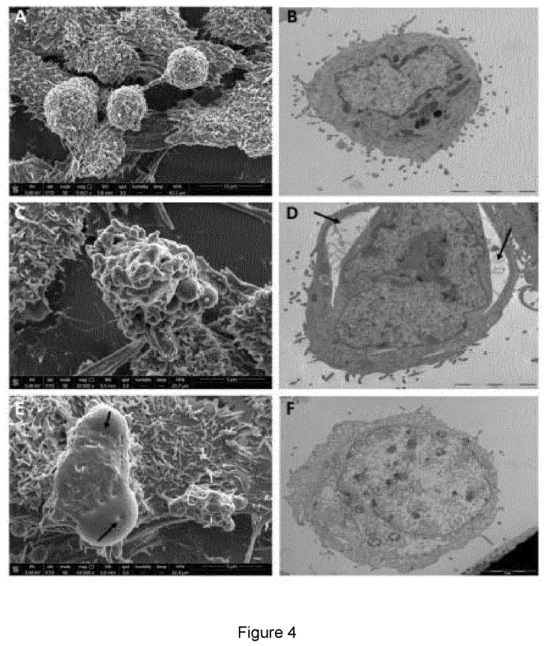

**Figure 4****.** (A, C, E) Scanning electron microscopy (SEM) analysis and (B, D, F) transmission electron microscopy analysis of colon cancer HCT-116 cell line. Microphotographs (A) and (B) represent HCT-116 control cells; (C, D, E, F) represent HCT116 *IdrB* after 3 days of gene induction. In comparison with the control, (C) apoptotic cells covered by a number of apoptotic bodies and (E) some cells with pores and "flat surfaces" (black arrow) are observed. At the ultrastructural level, (D) HCT-116 *IdrB* cells show a dilated endoplasmic reticulum (ER) and (F) disintegrated cells (black arrows). Scale bar = 5 µm.
**Figure 5****.** Different selected HCT-116 clones (H1, H2, and H3) transfected with the gene *hokD* (10x). In the absence of Dox, protein fluorescence is not observed. After 24 hours of administration with Dox, the fluorescence emitted by m-cherry and, therefore, the correct transfection and expression of the gene *hokD,* are shown.
**Figure 6****.** Different selected HCT-116 clones (L1, L2, and L3) transfected with the gene *IdrB* (10x). In the absence of Dox, protein fluorescence is not observed. After 24 hours of administration with Dox, the fluorescence emitted by m-cherry and, therefore, the correct transfection and expression of the gene *IdrB,* are shown
**Figure 7****.** Effects of the expression of the gene *hokD* on the HCT-116 H2 clone line (left 10x; right 20x).
**Figure 8****.** Effects of the expression of the gene *IdrB* on the HCT-116 L2 clone line (left 10x; right 20x).
**Figure 9****.** Different selected clones (H1, H2, and H3) of MCF-7 cells transfected with the gene *hokD* (10x). In the absence of Dox, protein fluorescence is not observed. After 24 hours of administration with Dox, the fluorescence emitted by m-cherry and, therefore, the correct transfection and expression of the gene *hokD,* are shown.
**Figure 10****.** Different selected MCF-7 clones (L1, L2, and L3) transfected with the gene *IdrB* (10x). In the absence of Dox, protein fluorescence is not observed. After 24 hours of administration with Dox, the fluorescence emitted by m-cherry and, therefore, the correct transfection and expression of the gene *IdrB,* are shown.
**Figure 11****.** Effects of the expression of the gene *hokD* on the MCF-7 H2 clone line (left 10x; right 20x).
**Figure 12****.** Effects of the expression of the gene *IdrB* on the MCF-7 L2 clone line (left 10x; right 20x).
**Figure 13****.** % fluorescence expression after induction of MCF7-L (A), MCF7-H (B), HCT-L (C), and HCT-H (D) with Dox for 10 days
**Figure 14****.** Cell proliferation curves for the HCT-116 line. It can be observed that the lines transfected with the genes *IdrB* and *hokD* in the presence of Dox cause a significant decrease in cell growth in comparison with the lines transfected without Dox and the control line.
**Figure 15****.** Cell proliferation curve for the MCF-7 line. It can be observed that the lines transfected with the genes *IdrB* and *hokD* in the presence of Dox cause a significant decrease in cell growth in comparison with the lines transfected without Dox and the control line.
**Figure 16****.** Effects of the expression of the genes *hokD* and *IdrB* on the HCT-116 cell line. The data showed a decrease in confluence.
**Figure 17****.** Effects of the expression of the genes *hokD* and *IdrB* on the MCF-7 cell line. The data showed a decrease in confluence.
**Figure 18****.** HeLa HCT-116 cells transfected with the gene *hokD* (10x). In the absence of Dox, protein fluorescence is not observed. After 24 hours of administration with Dox, the fluorescence emitted by z-green and, therefore, the correct transfection and expression of the gene *hokD,* are shown.
**Figure 19****.** Cell proliferation curve in the HeLa line. It can be observed that the lines transfected with the gene *hokD* in the presence of Dox cause a significant decrease in cell growth in comparison with the lines transfected without Dox and the control line.
**Figure 20**. (A) Growth rates of HCT-*hokD* tumor xenografts in SCID mice (the bars indicate the standard deviation of tumor volumes, n = 8 injected sites, * p <0.05, ** p <0.01 HCT-116 and HCT-*IdrB*-Dox; # p <0.05, ## p <0.01 HCT-116-Dox and HCT-*IdrB-*Dox). (B) Images representative of the fluorescent signal in Control and H+doxy resected tumors; and (C) comparison of HCT-116, HCT-116-dox, and HCT-*hokD*-Dox sizes, respectively.

### DESCRIPTION OF THE INVENTION

The inventors have selected two genes, *hokD* and *IdrB,* to study their behavior in eukaryotic cells, since they may be possible new candidates for being used in the suicide gene therapy in cancer given their many qualities in causing death in the host cell.

The main objective of the study was to investigate the therapeutic potential of the genes *hokD* and *IdrB,* using the MCF-7 breast cancer cell line and the HCT-116 colon cancer cell line. The inventors developed a new MCF-7 and HCT-116 cell line transfected with the genes *hokD* and *IdrB,* each one through the Tet-On 3G Inducible Expression System. This system is the gene expression that can be induced for mammalian cells. The target cells expressing transactivator protein Tet-On 3G and containing a gene of interest under the control of promotor PTRE3G will express high levels of the gene of interest, but only when they are cultured in the presence of doxycycline, which allows the controlled expression of the suicide gene in the tumor cell.

Induction of the expression of the genes *hokD* and/or *IdrB* in colon cancer and breast cancer caused changes in morphology and induced a clear antiproliferative effect. These results show a high therapeutic value. Furthermore, the characteristics of the Tet-On system allow the control of gene expression based on the addition or elimination of doxycycline.

The results suggest that the gene *hokD* and the gene *IdrB* reduce cell proliferation in transfected MCF-7 and HCT-116 cells, showing a cytotoxic activity that can be used as treatments for cancer in gene therapy.

Therefore, the present invention relates to the following aspects:

### Gene hokD

A first aspect of the present invention relates to an isolated RNA or DNA polynucleotide, hereinafter first polynucleotide of the invention, having an identity of at least 80%, preferably 85%, 90%, 95%, and more preferably 99% with the polynucleotide sequence of the gene *hokD* (SEQ ID NO: 1) for use in medicine, or alternatively, for use as a medicinal product. More preferably, the first polynucleotide of the invention is the gene *hokD,* with nucleotide sequence SEQ ID NO: 1

SEQ ID NO: 1

ATGAAGCAGCAAAAGGCGATGTTAATCGCCCTGATCGTCATCTGTTTAACCGTCATA
GTGACGGCACTGGTAACGAGGAAAGACCTCTGCGAGGTACGAATCCGAACCGGCCA
GACGGAGGTCGCTGTCTTCACAGCTTACGAACCTGAGGAGTAA

Alternatively, another **aspect** of the invention relates to an isolated RNA or DNA polynucleotide, hereinafter first polynucleotide of the invention, capable of being translated into an amino acid sequence comprising a peptide having an identity of at least 90% with the amino acid sequence of the protein HOKD (SEQ ID NO: 3). It more preferably relates to an isolated RNA or DNA polynucleotide capable of being translated into an amino acid sequence comprising a peptide having an identity of at least 95%, 96%, 97%, 98%, or 99% with the amino acid sequence of the protein HOKD, for use in medicine, or alternatively, for use as a medicinal product.

SEQ ID NO: 3
MKQQKAMLIALIVICLTVIVTALVTRKDLCEVRIRTGQTEVAVFTAYEPEE

Another **aspect** of the invention relates to a genetic DNA or RNA structure, hereinafter first genetic structure of the invention, comprising at least one of the following types of sequences:
a) a nucleotide sequence comprising at least the first polynucleotide of the invention for *in vitro* or *in vivo* transcription,
b) a nucleotide sequence corresponding to a gene expression system or vector comprising a polynucleotide of the invention, operatively linked with at least one promotor which directs the transcription of said nucleotide sequence, and with other sequences necessary or suitable for transcription and the suitable regulation thereof in time and place,
for use in medicine, or alternatively, for use as a medicinal product.

Another **aspect** of the invention relates to a cell, hereinafter first cell of the invention, comprising the first polynucleotide of the invention, or the first genetic structure of the invention.

Another aspect of the invention relates to a composition, hereinafter first composition of the invention, comprising:
I) the first polynucleotide of the invention, and/or
II) the first genetic structure of the invention,
for use in medicine, or alternatively, for use as a medicinal product.

In a preferred embodiment of this aspect of the invention, the first composition of the invention is a pharmaceutical composition. In another preferred embodiment of this aspect of the invention, the composition further comprises a pharmaceutically acceptable vehicle. In another preferred embodiment of the invention, the composition further comprises another active ingredient.

In a preferred embodiment, the first composition of the invention comprises another active ingredient, this active ingredient being an isolated RNA or DNA polynucleotide having an identity of at least 80%, preferably 85%, 90%, 95%, and more preferably 99% with the polynucleotide sequence of the gene *IdrB* (SEQ ID NO: 2). for use in medicine, or alternatively, for use as a medicinal product. More preferably, the polynucleotide of the invention is the gene *IdrB,* with nucleotide sequence SEQ ID NO: 2

SEQ ID NO: 2

ATGACGCTCGCGCAGTTTGCCATGACTTTCTGGCACGACCTGGCGGCACCGATCCT
GGCGGGAATTATTACCGCAGCGATTGTCGGCTGGTGGCGTAACCGGAAGTAA

Another **aspect** of the invention relates to the first polynucleotide of the invention, the first genetic structure of the invention, or the first composition of the invention, for the prevention, improvement, relief, or treatment of a proliferative disease. In a preferred embodiment, the proliferative disease is cancer. In another preferred embodiment, the proliferative disease is breast cancer. In another preferred embodiment, the proliferative disease is colon cancer.

### Gene IdrB

Another **aspect** of the present invention relates to an isolated RNA or DNA polynucleotide, hereinafter *second polynucleotide of the invention,* having an identity of at least 80%, preferably 85%, 90%, 95%, and more preferably 99% with the polynucleotide sequence of the gene *IdrB* (SEQ ID NO: 2) for use in medicine, or alternatively, for use as a medicinal product. More preferably, the second polynucleotide of the invention is the gene *IdrB,* with nucleotide sequence SEQ ID NO: 2

SEQ ID NO: 2

ATGACGCTCGCGCAGTTTGCCATGACTTTCTGGCACGACCTGGCGGCACCGATCCT
GGCGGGAATTATTACCGCAGCGATTGTCGGCTGGTGGCGTAACCGGAAGTAA

Alternatively, another **aspect** of the invention relates to an isolated RNA or DNA polynucleotide, hereinafter *second polynucleotide of the invention,* capable of being translated into an amino acid sequence comprising a peptide having an identity of at least 90% with the amino acid sequence of the protein LDRB (SEQ ID NO: 4). It more preferably relates to an isolated RNA or DNA polynucleotide capable of being translated into an amino acid sequence comprising a peptide having an identity of at least 95%, 96%, 97%, 98%, or 99% with the amino acid sequence of the protein LDRB, for use in medicine, or alternatively, for use as a medicinal product.

SEQ ID NO: 4
MTLAQFAMTFWHDLAAPILAGIITAAIVGWWRNRK

Another **aspect** of the invention relates to a genetic DNA or RNA structure, hereinafter *second genetic structure of the invention,* comprising at least one of the following types of sequences:
a) a nucleotide sequence comprising at least the second polynucleotide of the invention for *in vitro* or *in vivo* transcription,
b) a nucleotide sequence corresponding to a gene expression system or vector comprising a polynucleotide of the invention, operatively linked with at least one promotor which directs the transcription of said nucleotide sequence, and with other sequences necessary or suitable for transcription and the suitable regulation thereof in time and place,
for use in medicine, or alternatively, for use as a medicinal product.

Another **aspect** of the invention relates to a cell, hereinafter second cell of the invention, comprising the second polynucleotide of the invention, or the second genetic structure of the invention.

Another aspect of the invention relates to a composition, hereinafter second composition of the invention, comprising:
I) the second polynucleotide of the invention, and/or
II) the second genetic structure of the invention,
for use in medicine, or alternatively, for use as a medicinal product.

In a preferred embodiment of this aspect of the invention, the second composition of the invention is a pharmaceutical composition. In another preferred embodiment of this aspect of the invention, the composition further comprises a pharmaceutically acceptable vehicle. In another preferred embodiment of the invention, the composition further comprises another active ingredient.

In a preferred embodiment, the second composition of the invention comprises another active ingredient, this active ingredient being an isolated RNA or DNA polynucleotide having an identity of at least 80%, preferably 85%, 90%, 95%, and more preferably 99% with the polynucleotide sequence of the gene *hokD* (SEQ ID NO: 1), for use in medicine, or alternatively, for use as a medicinal product. More preferably, the polynucleotide of the invention is the gene *hokD,* with nucleotide sequence SEQ ID NO: 1

SEQ ID NO: 1

ATGAAGCAGCAAAAGGCGATGTTAATCGCCCTGATCGTCATCTGTTTAACCGTCATA
GTGACGGCACTGGTAACGAGGAAAGACCTCTGCGAGGTACGAATCCGAACCGGCCA
GACGGAGGTCGCTGTCTTCACAGCTTACGAACCTGAGGAGTAA

That is, the compositions of the present invention can be used together with other medicinal products in combined therapies. The other drugs may be part of the same composition or of another different composition, for administration at the same time or at different times.

Another **aspect** of the invention relates to the second polynucleotide of the invention, the second genetic structure of the invention, or the second composition of the invention, for the prevention, improvement, relief, or treatment of a proliferative disease. In a preferred embodiment, the proliferative disease is cancer. In another preferred embodiment, the proliferative disease is breast cancer. In another preferred embodiment, the proliferative disease is colon cancer.

### COMBINED PREPARATION OF THE INVENTION

Therefore, another **aspect** of the invention relates to a combined preparation, hereinafter combined preparation of the invention, comprising:
a) the first polynucleotide, the first genetic structure, and/or the first composition of the invention, or any combination thereof; and
b) the second polynucleotide, the first genetic structure, and/or the first composition of the invention, or any combination thereof.

Another **aspect** relates to the use of the combined preparation of the invention for use in medicine, or alternatively, for use as a medicinal product.

Another **aspect** relates to the combined preparation of the invention for simultaneous or sequential separately combined administration for the prevention, improvement, relief, or treatment of a proliferative disease. In a preferred embodiment, the proliferative disease is cancer. In another preferred embodiment, the proliferative disease is breast cancer. In another preferred embodiment, the proliferative disease is colon cancer.

Another **aspect** of the invention relates to a genetic DNA or RNA structure, hereinafter third genetic structure of the invention, comprising at least one of the following types of sequences:
a) a nucleotide sequence comprising at least the first polynucleotide and the second polynucleotide of the invention for *in vitro* or *in vivo* transcription,
b) a nucleotide sequence corresponding to a gene expression system or vector comprising the first polynucleotide and the second polynucleotide of the invention, operatively linked with at least one promotor which directs the transcription of said nucleotide sequence, and with other sequences necessary or suitable for transcription and the suitable regulation thereof in time and place,
for use in medicine, or alternatively, for use as a medicinal product.

Another **aspect** relates to the third genetic structure of the invention for the prevention, improvement, relief, or treatment of a proliferative disease. In a preferred embodiment, the proliferative disease is cancer. In another preferred embodiment, the proliferative disease is breast cancer. In another preferred embodiment, the proliferative disease is colon cancer.

The term "medicinal product", as it is used herein, refers to any substance used for the prevention, diagnosis, relief, treatment, or healing of diseases in humans and animals.

As it is used herein, the term "active ingredient", "active substance", "pharmaceutically active substance", or "pharmaceutically active ingredient" means any component that potentially provides pharmacological activity or another different effect in the diagnosis, healing, mitigation, treatment, or prevention of a disease, or affects the structure or function of the body of humans or other animals. The term includes those components which promote a chemical change in the preparation of the drug and are present therein in an intended modified form that provides the specific activity or effect.

It should be emphasized that the term "combined preparation", also referred to as a "juxtaposition", herein means that the components of the combined preparation do not need to be present bound to one another, for example in a true composition, in order to be available for their combined, separate, or sequential application. Therefore, the expression "juxtaposed" means that it is not necessarily a true combination given the physical separation of the components.

The compositions or the combined preparation of the invention can be administered to the subject who suffers from said pathologies by means of any of the following routes: intraperitoneal, intravenous, intramuscular, subcutaneous, intrathecal, intraventricular, oral, enteral, parenteral, intranasal, or dermal. In a preferred embodiment, the routes of administration are, preferably, the intravenous route and the oral route.

Both the compositions of the present invention as well as the combined preparation or the pharmaceutical forms of the invention can be formulated for administration to an animal, and more preferably to a mammal, including humans, in a variety of forms known in the state of the art. In that sense, they can be, without limitation, in a sterile aqueous solution or in biological fluids, such as serum. The aqueous solutions may or may not be buffered and have additional active or inactive components. The additional components include salts for modulating ionic strength, preservatives including but not limited to antimicrobial agents, antioxidants, chelators, and similar agents, and nutrients including glucose, dextrose, vitamins, and minerals. Alternatively, the compositions can be prepared for administration in solid form. The compositions can be combined with several inert excipients or vehicles, including but not limited to binders such as microcrystalline cellulose, tragacanth gum, or gelatin; excipients such as starch or lactose; dispersing agents such as alginic acid or corn starch; lubricants such as magnesium stearate; glidants such as colloidal silicon dioxide; sweetening agents such as sucrose or saccharine; or flavoring agents such as mint or methyl salicylate.

Such compositions or combined preparations and/or their formulations can be administered to an animal, including a mammal and, therefore, to humans, in a variety of ways, including but not limited to intraperitoneal, intravenous, intramuscular, subcutaneous, intrathecal, intraventricular, oral, enteral, parenteral, intranasal, or dermal.

The dosage for obtaining a therapeutically effective amount depends on a number of factors, such as for example, the age, weight, sex, tolerance, etc., of the mammal. In the sense used in this description, the expression "therapeutically effective amount" refers to the comprised amount of active ingredient or ingredients of the invention producing the desired effect, and it will generally be determined, among other causes, by the characteristics that are typical of said prodrugs, derivatives, or analogues, and the therapeutic effect to be achieved. The "adjuvants" and "pharmaceutically acceptable vehicles" that can be used in said compositions are the vehicles known to those skilled in the art.

Therefore, the administered amount of a compound of the present invention will depend on the relative efficacy of the chosen compound, the seriousness of the disease to be treated, and the weight and/or other characteristics of the patient. However, compositions or the combined preparation of this invention will be administered, for example, but without limitation, one or more times per day, for example 1, 2, 3, or 4 times daily, with a total dose between 0.1 and 1000 mg/kg/day. It is important to take into account that, depending on the age and on the condition of the patient, it may be necessary to introduce variations in the dose, as well as modifications in the route of administration.

Throughout the description and claims the word "comprises" and variants thereof do not intend to exclude other technical features, additives, components, or steps. For those skilled in the art, other objects, advantages, and features of the invention will be inferred in part from the description and in part from putting the invention into practice. The following examples and drawings are provided by way of illustration and are not intended to be limiting of the present invention.

### EXAMPLES OF THE INVENTION

### Materials and methods

### Cell culture and drugs

HCT-116 human colorectal carcinoma cell line, MCF-7 human breast carcinoma cell line, and HeLa cervical cancer cell line were obtained from Biobanco (Andalusian Public Health System, Granada). The cells were cultured in Dulbecco's Modified Eagle medium (DMEM) (Sigma, St. Louis, MO, USA), supplemented with 10% fetal bovine serum (FBS) and 1% penicillin/streptomycin (P/S) (Sigma-Aldrich), under air containing 5% CO₂ in an incubator at 37°C.

For the selection of transfected clones, the growth medium was supplemented with geneticin (800 µg/ml) and puromycin (1.5 µg/ml). A growth medium supplemented with geneticin (200 µg/ml) and puromycin (0.75 µg/ml) was used to maintain the selected colonies. To induce gene expression, doxycycline was used (Dox, 3 µg/ml). All the antibiotics were provided by Clontech Laboratories, Inc. (UNITED STATES).

### Production of stable inducible cell clones.

The Tet-On gene expression system (Clontech Laboratories, Inc. (USA)) was used to express the genes *hokD* and *IdrB.* The HCT-116, MCF-7, and HeLa cells were transfected initially with pTet-On 3G (Xfect Polymer, Clonthech, USA), and the successfully transfected clones were selected by geneticin resistance (800 µg/ml). These clones were transfected with PTRE3G-hok and PTRE3G-ldr, selected for geneticin (200 µg/ml) and puromycin (1.5 µg/ml). All the clones were cultured in the presence of doxycycline (Dox, 3 µg/ml, 24 h) to induce expression of the genes *hokD* (HCT-H, MCF-H, and HeLa-H) and *IdrB* (HCT-L and MCF-L), which was detected by fluorescence.

### Microscopy analysis

Gene expression was confirmed in transfected HCT-116 and MCF-7 with excitation at 587 nm. Fluorescent microscopy analysis was performed with a Nikon Eclipse Ti (Nikon Instruments Inc. NY, USA).

### Antibiotic tolerance study

The tolerance curves for doxycycline at different concentrations were first assessed. HCT-116 cells (1,000 cells per well) and MCF-7 cells (1,500 cells per well) were seeded in a 96-well plate. After 2, 4, and 6 days for the induction with doxycycline (using different concentrations of 2 µg/ml to 6 µg/ml), 10 µl of solution CCK-8 were added to each well of the plate and incubated at 37°C for 3 h. Absorbance at 450 nm was then measured using a Thermo Scientific Multiskan microplate reader.

To assess the antibiotic tolerance curves for different concentrations of geneticin and puromycin, HCT-116 cells were seeded in a 6-well plate (50,000 cells per well). After 2, 4, and 6 days for the selection of induction with geneticin (concentration of 500 µg/ml to 800 µg/ml) and puromycin (concentration of 0.5 µg/ml to 2 µg/ml), the cells were examined using a phase contrast microscope to assess the possible effects.

### Fluorescence study

Fluorescence of the red fluorescent protein m-cherry was determined by means of FACSan flow cytometry analysis (Becton Dickinson, San Jose, CA), with a maximum excitation of 587 nm and a maximum emission of 601 nm (Clontech Laboratories, Inc. (USA)).

### Proliferation and confluence assay

The HCT-116 and MCF-7 cancer cell lines were kept in Dulbecco's Modified Eagle Medium (DMEM) without phenol red (Sigma, St. Louis, MO, USA), supplemented with 10% fetal bovine serum (FBS), 100 µg/ml of penicillin and 100 µg/ml of streptomycin. The cancer cells were seeded in 96-ViewPlate^{™} microplates (transparent bottom, white), seeding 1500 cells for the MCF-7 cell line per well and 1000 cells for the HCT-116 cell line per well. The plates were incubated at 37°C in the presence of 5% CO₂.

24 h after seeding, 3 µg/ml of Dox were added in different wells. The assay plate was read in the EnSight system for 15 days using the brightfield imaging mode. The plates were kept under controlled conditions (5% CO₂, 37°C, and 90% humidity) between each reading, and imaging was carried out at 37°C using the temperature control function of the EnSight reader, which eliminates the possible water condensation effects. The times of the reading cycle were <5 min per plate. The images were analyzed using Kaleido^{™} data acquisition and analysis software, and the derived results were fitted to a 4 parameter logistic curve and plotted using TIBCO Spotfire^{®}.

*Analysis of the cell cycle and measurement of SubG1 cells through flow cytometry conditions.*

Cell cycle phases (G0/G1, S, or G2/M) were characterized according to the cell DNA content. The fluorescent dye propidium iodide (PI) (Sigma, USA) binds strongly to DNA at a ratio of 1:1; therefore, the DNA content of the cell cycle phases were reflected by means of variable PI fluorescent intensities.

The cells in monolayer culture were collected, washed twice with PBS, and fixed in ice cold 70% ethanol at 4°C. The cell pellets were washed twice with PBS, resuspended in a DNA extraction solution (pH = 7.8) consisting of acetic acid (0.1 M) and sodium phosphate dibasic anhydrous (0.2 M) in PBS, and incubated for 15 minutes at 37°C. The cells pelleted and were washed once more with PBS and resuspended in PI/RNase/PBS solution (100 mg/ml of PI and 10 mg/ml of RNase) for 30 minutes at 37°C in darkness. The percentage of cells in phases subG1, G0/G1, S, and G2/M was determined by means of (fluorescence-activated cell sorting) flow cytometer FACSCalibur (BD Biosciences, USA).

### Transmission electron microscopy (TEM)

The adherent HCT-116 cell lines induced with Dox (3 µg/ml) for 6 and 3 days were fixed with a solution of 2.5% glutaraldehyde in 0.1 M sodium cacodylate buffer (pH 7.4; Sigma) for 2 hours. The pellet that contained dead cells and debris, as well as monolayer, were later fixed with 1% osmium tetroxide in 0.1 M sodium cacodylate buffer (Sigma) and dehydrated in ethanol. The cells were separated from the culture of the culture vessel by means of rapid treatment with propylene oxide and embedded in Epon 812 (EMS, Washington, USA). After polymerization, the plastic was removed, and ultrathin sections were cut parallel and perpendicular to the surface of the flask. The sections were contrasted with uranyl acetate-lead citrate and examined under a Carl Zeiss EM 902 transmission electron microscope (TEM).

### Scanning electron microscope (SEM)

The cells were cultured in sterile slipcovers and induced for 3 and 6 days with Dox (3 µg/ml). They were fixed for 2 hours at 40°C with 2.5% glutaraldehyde in 0.05 M cacodylate buffer, pH 7.4, containing 0.7 M KCI, 1.24 mM CaCl₂, and 1.24 mM MgCl₂. They were then washed and treated for 1 hour with 1% tannic acid in 0.1 M cacodylate buffer at 4°C and briefly washed again in the same buffer. The fixed cells were concentrated again by centrifugation in 1% agarose and were later fixed in 2% OsO₄. The cell cultures were dehydrated in amyl acetate/ethanol followed by a critical point of drying and coating with gold. A Hitachi S-800 scanning electron microscope (Hitachi, Tokyo) was used for the observations.

### Statistical analysis

The results presented in this paper were performed in triplicate and compared with the Student's T-test. All the data is expressed as the mean ± SD. The differences were considered statistically significant with a value P ≤ 0.05.

### Results

### Detection and effects of the expression of the genes hokD and IdrB on the HCT-116 cell line

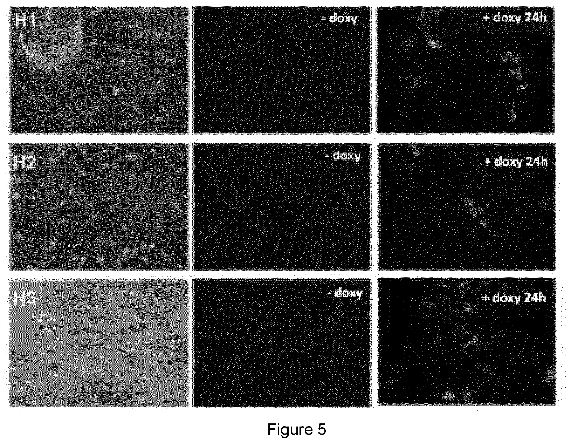

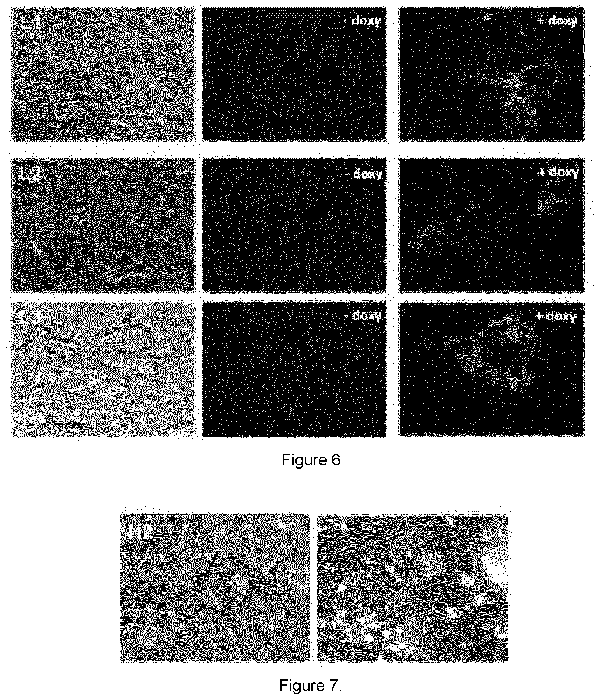

The cells were transfected with genes of interest and their expression induced with Dox (3 µg/ml). 24 hours after induction, detection of the expression of the gene was studied by means of fluorescence microscopy through the fluorophore m-cherry, which is found in promotor PTRE3G, and its expression, therefore, acts as an indicator of the expression of the gene of interest which produces a red protein. Three clones for each of the genes where then selected (Figures 5 and 6).

After 6 days of inductions with Dox, it was observed that the gene expression transformed the shapes of the cells from polygonal to circular and caused the cells to shrink. Furthermore, a reduction in the number of cells was observed, and a number of floating cells were detected. Two clones of each transfected gene were selected to continue with further studies (Figure 7).

### Detection and effects of the expression of the genes hokD and IdrB on the MCF-7 cell line

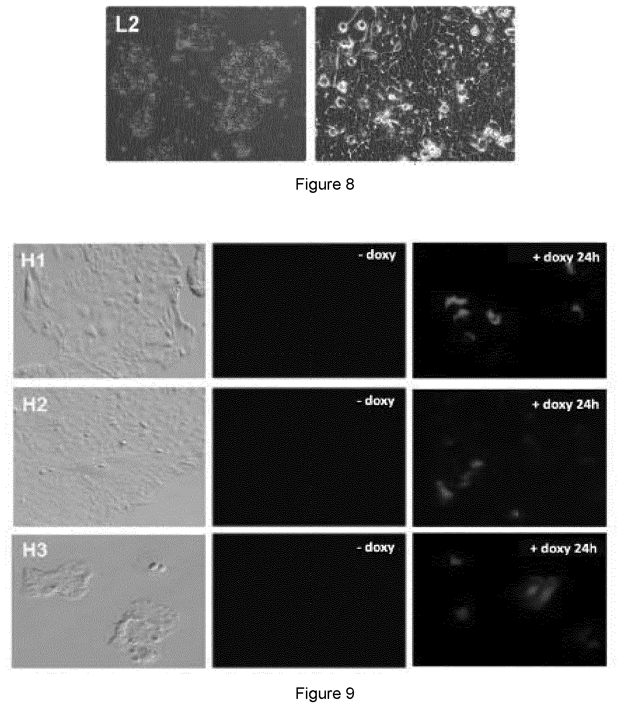

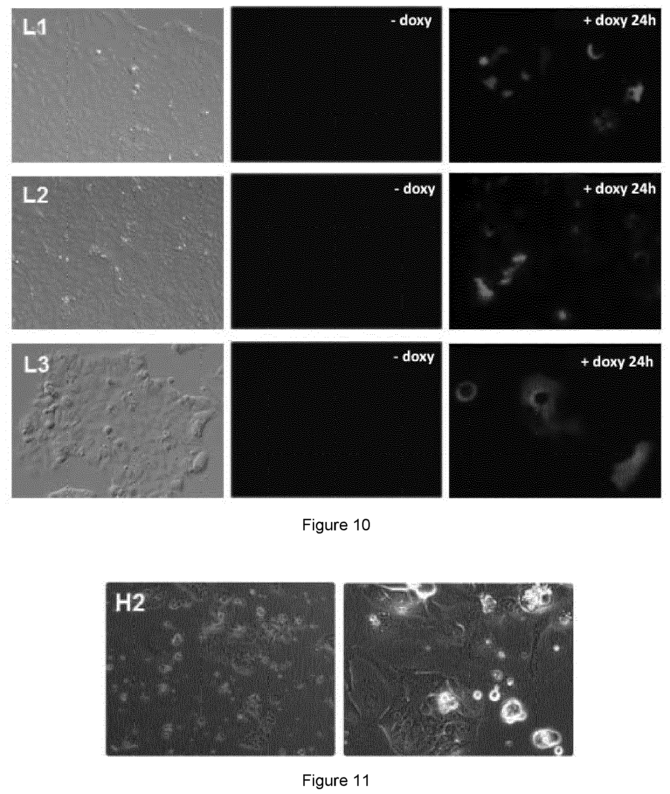

Like the previous HCT-116 line, the morphology of MCF-7 cells was examined for the first time under normal conditions using a phase contrast microscope. Similarly to the previous cell line, the cells were transfected with genes of interest and their expression induced with Dox (3 µg/ml). 24 hours later, the expression of the gene was detected by fluorescence microscopy through the fluorophore m-cherry, which is found in promotor PTRE3G, and its expression, therefore, acts as an indicator of the expression of the gene of interest which produces a red protein. Three clones for each of the genes where then selected (Figures 9 and 10).

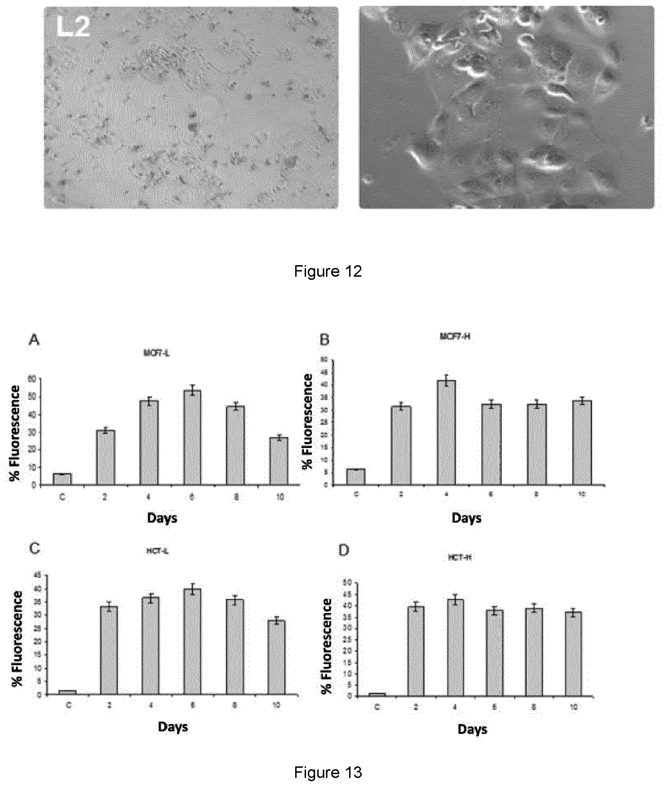

After 6 days of inductions with Dox, it was observed that the gene expression transformed the shapes of the cells which lost its well-defined morphology, became round and spread out. Furthermore, a reduction in the number of cells was observed, and a number of floating cells were detected. Two clones of each transfected gene were selected to continue with further studies (Figures 11 and 12).

### Fluorescence assay

The percentage of fluorescence after induction with doxycycline was studied. The data shows that the administration of Dox every two days produced constant levels of fluorescence in both cell lines (Figure 13). Therefore, the constant expression of fluorescence also functions as an indicator of gene expression.

### Study of cell proliferation in lines transfected with genes hokD and IdrB

Once it was verified that Dox does not affect cell growth, the proliferation study was performed in both lines, HCT-116 and MCF-7, transfected with the genes *hokD* e *IdrB,* both in the presence and in the absence of Dox, in addition to the study of the control line. For this, Dox was administered every 48 hours to maintain the effect of gene expression. The data was obtained using the EnSight system in brightfield imaging mode.

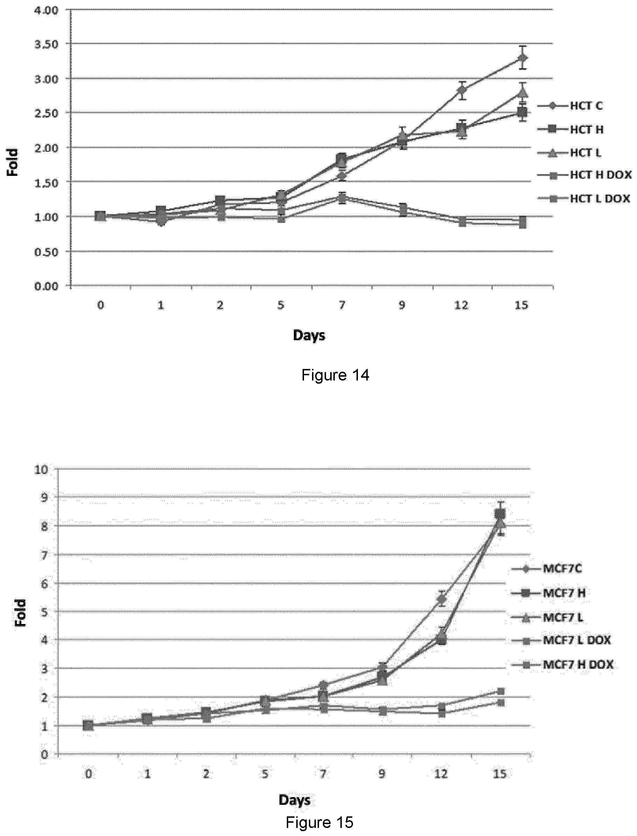

In the HCT-116 cell line transfected with the genes *IdrB* and *hokD,* the cell inhibition rate was observed when Dox was administered. An exponential increase in the proliferation rate in the parent cell line (without transfection and absence of Dox), as well as in the cell lines transfected with both genes but without induction with Dox, was observed. However, the HCT-H cell line in the presence of Dox sustains a drop in cell growth of 48%, 70.85%, and 71.21% after 9, 12, and 15 days of treatment, respectively. Similarly, the HCT-L line obtains a drop in cell growth of 51.18% after 9 days of treatment, and 71.45% and 71.82% after 12 and 15 days, respectively (Figure 14).

A similar effect occurs in the MCF-7 cell line. In the presence of Dox, a significant drop in cell growth in lines transfected with genes *hokD* and *IdrB* was observed. However, lines transfected in the absence of Dox and the control line grow as normal. The data shows that the MCF-L cell line induces 38.10% inhibition of proliferation after 9 days of treatment, and 69% and 72.80% after 12 and 15 days, respectively. Similarly, the MCF-H cell line sustains a drop in cell growth of 33.93% after 9 days of treatment, and 73.66% and 77.51% after 12 and 15 days, respectively (Figure 15).

### Study of confluence in lines transfected with genes hokD and IdrB

Cell confluence is also determined using the EnSight system in brightfield imaging mode. The degree of confluence is defined as the percentage of the area of the image covered with cells. This mode does not require any staining for the cell nuclei or cytoplasm.

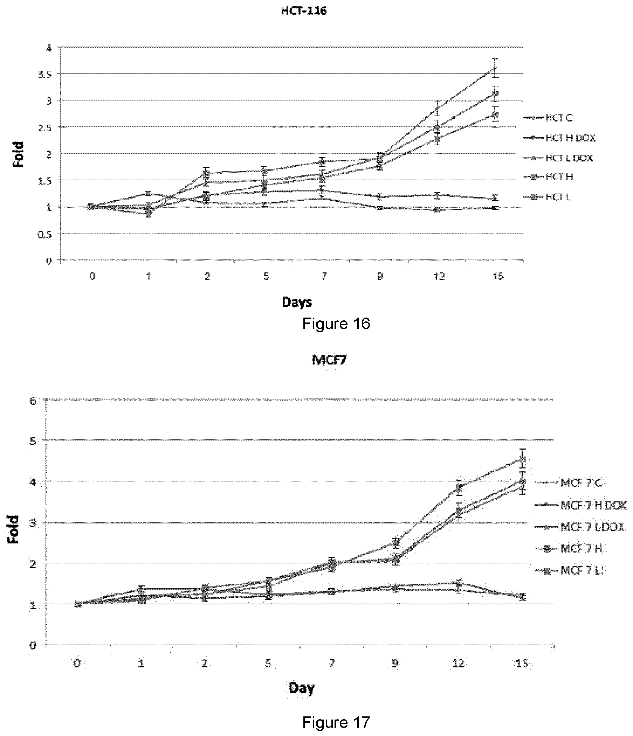

In both cell lines transfected with the gene *IdrB* and *hokD* with the induction of gene expression, a drop in confluence in comparison with the control line and the line transfected without inducing gene expression (in the absence of doxycycline) was shown. The HCT-H cell line sustains a drop in confluence of 38.02% after 9 days of treatment, and 57.54% and 67.77% after 12 and 15 days, respectively. Similarly, the HCT-L cell line obtains a drop in confluence of 48.95% after 9 days of treatment, and 67.01% and 75.77% after 12 and 15 days, respectively (Figure 16).

In MCF-H, the cell line showed a drop in confluence of 33.98% after 9 days of treatment, and 57.59% and 68.77% after 12 and 15 days, respectively. Similarly, the MCF-L cell line obtains a drop in confluence of 31.06% after 9 days of treatment, and 51.98% and 70.54% after 12 and 15 days, respectively (Figure 17).

### Genes hokD and IdrB induce apoptosis in HCT-116 cells

Apoptotic cell death can be determined by means of flow cytometry through staining with propidium iodide (PI) which is determined to determine the Sub-G1 peak. In HCT-116 *hokD,* Dox observed an increase in apoptotic cells over the time of gene induction. Days 3 and 6 of gene expression did not show significant modifications in relation to the control culture. In contrast, after 9 and 12 days of induction there was a significant increase in the percentage of apoptotic cells with 17.23% and 62.86%, respectively, whereas this percentage without induced cells was 2.14% (Figure 1A). Similar trends were observed for induced HCT-116 *IdrB* cells at the same time points, where the percentage of apoptotic cells was 53.81% and 94.01% for the induction of 9 and 12 days, respectively (Figure 1B).

### Cell cycle: hokD causes cell cycle arrest in phase G2/M whereas IdrB increases phase S

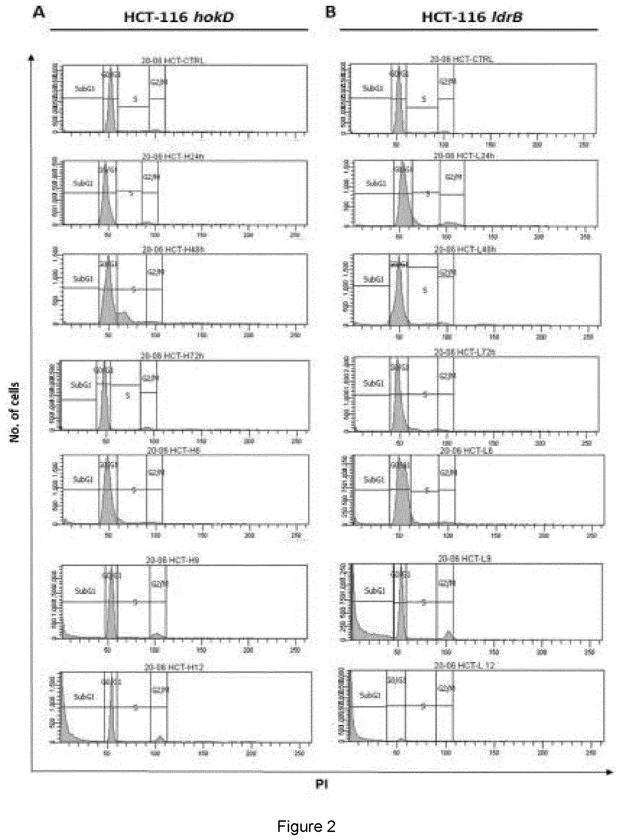

The distribution of the cell cycle (Figure 2) depending on the DNA content was examined by flow cytometry, discriminating cell phases G0/G1, S, G2/M (Figures 1C, D). HCT-116 *hokD* and HCT-116 *IdrB* cells were induced at different time points with Dox for 12 days. The control cell culture contained 88.56% of G1/G0 cells, 6.30% of cells in phase S, and 5.14% of cells in phase G2/M (Figures 1C, D).

48 h after starting the expression of *hokD,* the HCT-116 cells showed an increase in the population in phase S (21.85%) and a drop in the number of G0/G1 cells (74.21%). Furthermore, after 9 (10.37%) and 12 (14.58%) days of induction, the percentage of cells in phase G2/M increases (Figure 1C). In contrast, induction of the gene HCT-116 *IdrB* caused an increase in the proportion of cells in phase S after 9 (11.04%) and 12 (20.89%) days in comparison with the control (Figure 1D).

### Ultrastructural changes in HCT-116 cells expressing genes hokD and IdrB

SEM and TEM microscopy was used to examine the surface and the intracellular morphology in the HCT-116 control and in the transfected cells after 3 days of induction of the genes *hokD* and *IdrB.*

Previously described as pore forming proteins [34,39], there are also pores in the plasma membrane of transfected HCT-116 cells. Furthermore, the transfected cells showed a "flat cell surface" due to the loss of microvillus and filopodium structures (Figures 3C, E for the gene *hokD* and Figures 4C, E for the gene *IdrB)* in comparison with the controls (Figures 3A and 4A). Furthermore, a number of apoptotic bodies are observed on the cell surface (Figures 3C and 4C), which supports the above results (see section 2.1) in which apoptosis increases in the suicide genes *hokD* and *IdrB* expressing genes.

With respect to the ultrastructural changes in comparison with the control cells (Figures 3A and 4A), HCT-116 *hokD* showed dilated mitochondria (Figures 3D, F), whereas HCT-116 *IdrB* exhibited a significant dilation of the endoplasmic reticulum (ER) (Figures 4D, F). Furthermore, both the expression of *hokD* and the expression of *IdrB* induced cell disintegration (Figures 3G and 4F).

The results show that the expression of these genes induced a rapid drop in cell proliferation in colon cancer (71.21% for the gene *hokD* and 71.82% for the gene *IdrB)* and breast cancer (72.80% for the gene *hokD* and 77.51% for the gene *IdrB).* This data is consistent with the observed drop in confluence as a result of the toxic effect of gene expression. Furthermore, the technology used to study cell proliferation is part of the new imaging methods offered by the EnSight system. This imaging technology is an ideal instrument for determining important cytometric parameters, such as the number of cells and the level of confluence, without the use of fluorescent dyes which may sometimes be expensive. It presents a non-destructive reading which allows data about the kinetics of the possible toxic effects to be collected. In addition to studying parameters such as confluence, morphological characteristics such as "cell roughness" were also extracted, providing useful secondary readings complementing the basic data [31,32]. It would be necessary to determine whether the drop in cell proliferation and confluence is due to apoptosis phenomena or cell cycle arrest. For example, one of the characteristics that takes part in the development of colon cancer and the formation of metastasis is resistance to apoptosis phenomena.

One of the main challenges for gene therapy is the appropriate expression of the therapeutic gene (suicide gene) in the target cells (tumor cells). To that end, being able to control the experimental dose is needed to obtain a controlled expression of the therapeutic gene. Therefore, there has to be a balance between expression of the therapeutic protein to obtain successful treatment and non-excessive production to avoid possible toxicity.

To obtain a safe treatment, it is necessary to verify the therapeutic induction of the gene. Therefore, several eukaryotic gene promotors have been tested for administering genes in a controlled manner. Some of these promotors include steroid hormones, heavy metals, oxygen, and some physical stimuli such as radiation [33-35]. However, most of these promotors have certain limitations in clinical applications. One of the potential risks is that because they are mammalian gene promotors, the transcription of endogenous genes may be affected. Furthermore, the inducers of these promotors are often endogenous molecules such as, for example, oxygen or hormones, such that the levels are hard to control or modulate in a safe manner. Another limitation includes possible side effects or an increase in the toxicity potential due to the inducer. Therefore, a system which allows safe control of gene expression in response to the action of inducible agents is needed.

The tetracycline-based regulation system offers said controlled regulation of the expression of the target gene in response to the administration of doxycycline (Dox) [36,37]. The Tet-On system is characterized by two regulating elements: the Tet-On transactivation proteins which bind specifically to the inducible promotor PTRE3G and activate the transcription of the gene of interest, but only in the presence of doxycycline. Inducible promotor PTRE3G provides a very low baseline expression and a high maximum expression after induction [38]. Therefore, gene expression can be suppressed in the absence of Dox. Furthermore, another advantage provided by this system is the low toxicity derived from tetracyclines, such as doxycycline, for example, which means that the doses of these antibiotic concentrations do not produce adverse effects [39,40]. For all these reasons, the system is a therapeutic tool applicable to clinical practice in gene therapy.

The study of apoptosis was investigated at different time points of the induction with Dox. A clear increase in apoptosis for both genes can be found after 9 days (17.23% for the gene *hokD* and 53.81% for the gene *IdrB)* and 12 days (62.86% for the gene *hokD* and 94.01% for the gene *IdrB)* of gene expression. Furthermore, the genes *hokD* and *IdrB* are known as pore forming toxins in bacterial cells [34]. Both belong to different *E. coli* type I TA modules (hok/sok and Idr/rdl families), for which the translation of toxins is inhibited by antisense sRNA antitoxin. The transmembrane domain of these toxins is expected to localize by interacting with other proteins in the cell membrane or periplasm and/or localize to the internal membrane, where the oligomer form of the toxin leads to pore-like structures and membrane permeabilization [39]. According to the effect of the toxin on the membrane of prokaryotic cells, the same pore forming capacity in the plasma membrane of human HCT-116 cells examined by SEM microscopy is found. Furthermore, a characteristic cell surface in the transfected cells is observed, with a loss of microvilli and filopodia that produced cells with a flat appearance, as well as a number of apoptotic bodies. Apoptosis as a mechanism of action of the genes *hokD* and *IdrB* was supported by TEM microscopy. Apoptosis can be triggered by different biochemical changes, such as DNA and protein degradation, membrane alteration and detection by phagocytic cells, and/or caspase stimulation [46]. It should be highlighted that these ultrastructural findings revealed dilated mitochondria in HCT-116 *hokD* cells, which is a distinctive sign of the intrinsic mitochondria-mediated apoptotic pathway. Interruption of the mitochondrial membrane potential leads to the release of apoptogenic factors, such as cytochrome c, which ultimately initiate the stimulation of those caspases that play a key role in apoptotic processes [9,12]. Furthermore, induced HCT-116 *IdrB* cells have a dilated ER that can trigger ER stress. Prolonged ER stress can cause the induction of cell death mediated by the C/EBP homologous protein (CHOP) [47]. Further investigation would be needed to assess the molecular mechanism whereby the genes *hokD* and *IdrB* expressed by HCT-116 undergo apoptosis.

Furthermore, analysis of the cell cycle allows cell cycle phases G0/G1, S, G2/M to be assessed. The distribution of the cell cycle phase was checked 1, 2, 3, 6, 9, and 12 days after induction of the gene Dox. The results revealed a notable accumulation of cells in phase S day 2 after induction of Dox; Furthermore, phase G2/M was notably higher after 9 (10.37%) and 12 (14.58%) days in comparison with the control (5.14%). A more obvious effect of the cell cycle was caused by the gene *IdrB,* where the percentage of cells in phase S was two-fold (11.04%) and three-fold (20.89%) higher after 9 and 12 days of gene induction, respectively, in comparison with the control (6.30%). Therefore, the hypothesis that the expression of *hokD* causes cell cycle arrest in phase S, whereas the gene *IdrB* does the same in phase G2/M in HCT-116 cells can be considered. In the eukaryotic cell, the checkpoint mechanisms of phase S control defective DNA replication or defective DNA, stress resulting from replication, and alterations in the chromatin structure [48]. Furthermore, cells with abnormal DNA can cause cell cycle arrest at checkpoint G2/M through the induction of the p53 pathway, which can lead to the start of repair or apoptosis. Although the usefulness of the genes *hokD* and *IdrB* has not previously been analyzed, other *E. coli* toxins have been tested to see their effect on cancer cells. For example, *E. coli* verotoxin 1 demonstrated an antiproliferative effect and detained HCT-116 colon cancer cells in phase S [49]. In the same way, the gene gef, which encodes a toxic protein operating in a manner similar to hok [50], together with apoptin (a small protein encoded by chicken anemia virus) induced apoptosis in colon cancer cells and caused cell cycle arrest in G2/Phase M [26].

### Detection and effects of expression of the gene hokD on the HeLa cell line

Similarly to the HCT and MCF-7 cell lines, the same proliferation study was carried out on the HeLa human cervical cancer cell line.

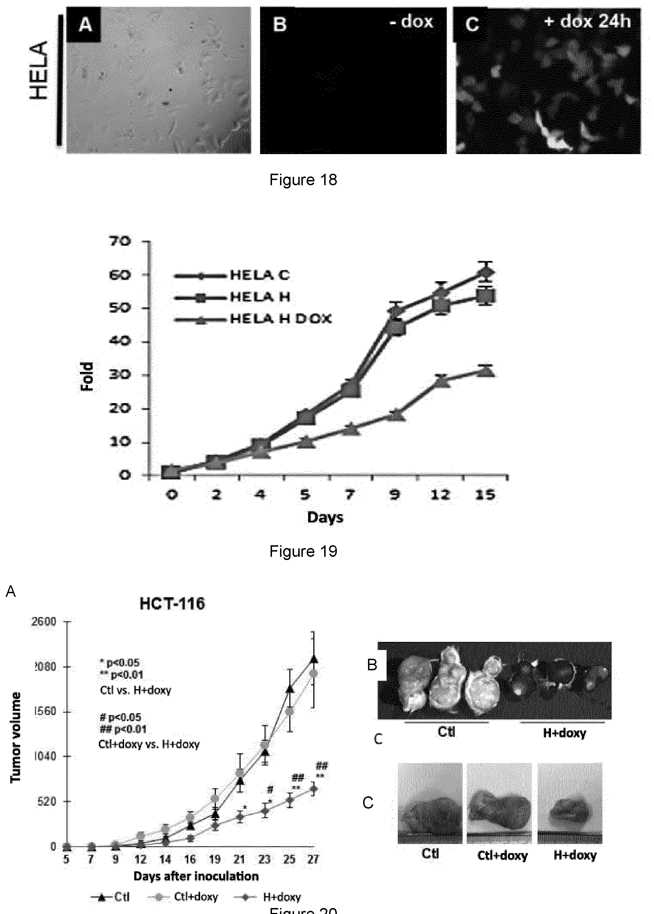

The HeLa line cells were transfected with *hokD* and their expression induced with Dox (3 µg/ml). 24 hours later, the expression of the gene was detected by fluorescence microscopy through the fluorophore z-Green (C), which is found in promotor PTRE3G, and its expression, therefore, acts as an indicator of the expression of the gene of interest which produces, in this case, a green protein (Figure 18).

HeLa cells were less sensitive to *hokD* that the other lines were, although they showed a reduction in cell growth of 42.6% after 15 days of treatment (Figure 19).

### Study of inhibition of the growth of tumor xenografts in nude mice using the gene hokD

Following the criteria and recommendations of the University of Granada Ethics Committee, *in vivo* testing was conducted only with colon cancer lines to minimize the amount of mice used, thereby avoiding the unnecessary suffering of animals.

First, the therapeutic efficacy of *hokD*-mediated suicide gene therapy using heterotopic tumor xenografts established by the HCT-116 and HCT-hokD cell lines was assessed. HCT-116 parent cells and HCT-hokD cells were injected subcutaneously into BALB/c mice. Once the tumors were felt, Dox was administered (100 mg/kg) by means of intraperitoneal injection, established that day as day 0. A third group of mice with HCT-116 tumors without the administration of Dox was used as the control.

The obtained results demonstrated that Dox alone had no effect on tumor proliferation. Therefore, no significant differences in the growth of HCT-116 and HCT-116-Dox tumors were observed. However, the proliferation of HCT-hokD-Dox tumors was significantly inhibited by expression of the gene *hokD* after 21 days (54.83%) and reached 70.68% in 25 days (Figure 20, a). This antitumor effect was maintained until the end of the experiment, maintaining an average tumor volume about 70% lower in comparison with the control group (Figure 20, A, C).

The *in vivo* images of resected tumors confirmed expression of the gene *hokD* in HCT-hokD-Dox tumors as shown in the representative images of the fluorescent signal (Figure 20, B).

### Literature:

1. Siegel, R. L.; Miller, K. D.; Jemal, A. Cancer statistics. 2016, 66, 7-30.
2. Manuscript, A. NIH Public Access. 2013, 2012.
3. Vannucci, L.; Lai, M.; Chiuppesi, F.; Ceccherini-Nelli, L.; Pistello, M. Viral vectors: a look back and ahead on gene transfer technology. New Microbiol. 2013, 36, 1-22.
4. Amer, M. H. Gene therapy for cancer: present status and future perspective. Mol. Cell. Ther. 2014, 2, 27.
5. Karjoo, Z.; Chen, X.; Hatefi, A. Progress and problems with the use of suicide genes for targeted cancer therapy. Adv. Drug Deliv. Rev. 2016, 99, 113-128.
6. Ortiz, R.; Melguizo, C.; Prados, J.; Alvarez, P. J.; Caba, O.; Hita, F.; Aránega, A. New Gene Therapy Strategies for Cancer Treatment: A Review of Recent Patents. 2012, 297-312.
7. Greco, O.; Dachs, G. U. Gene directed enzyme/prodrug therapy of cancer: Historical appraisal and future prospectives. J. Cell. Physiol. 2001, 187, 22-36.
8. Wang, J.; Lu, X.-X.; Chen, D.-Z.; Li, S.-F.; Zhang, L.-S. Herpes simplex virus thymidine kinase and ganciclovir suicide gene therapy for human pancreatic cancer. World J. Gastroenterol. 2004, 10, 400-3.
9. Kuriyama, S.; Kikukawa, M.; Masui, K.; Okuda, H.; Nakatani, T.; Akahane, T.; Mitoro, A.; Tominaga, K.; Tsujinoue, H.; Yoshiji, H.; Okamoto, S.; Fukui, H.; Ikenaka, K. Cancer gene therapy with HSV-tk/GCV system depends on T-cell-mediated immune responses and causes apoptotic death of tumor cells in vivo. Int. J. Cancer 1999, 83, 374-380.
10. Portsmouth, D.; Hlavaty, J.; Renner, M. Suicide genes for cancer therapy. Mol. Aspects Med. 2007, 28, 4-41.
11. Bentires-Alj, M.; Hellin, A. C.; Lechanteur, C.; Princen, F.; Lopez, M.; Fillet, G.; Gielen, J.; Merville, M. P.; Bours, V. Cytosine deaminase suicide gene therapy for peritoneal carcinomatosis. Cancer Gene Ther. 2000, 7, 20-6.
12. Dachs, G. U.; Tupper, J.; Tozer, G. M. From bench to bedside for gene-directed enzyme prodrug therapy of cancer. Anticancer. Drugs 2005, 16, 349-359.
13. Lin, B.; Gao, A.; Zhang, R.; Ma, H.; Shen, H.; Hu, Q.; Zhang, H.; Zhao, M.; Lan, X.; Liu, K. Use of a Novel Integrase-Deficient Lentivirus for Targeted Anti-Cancer Therapy With Survivin Promoter-Driven Diphtheria Toxin A. Medicine (Baltimore). 2015, 94, e1301.
14. Kim, H. P.; Frankel, A. E.; Hogge, D. E. A diphtheria toxin interleukin-3 fusion protein synergizes with tyrosine kinase inhibitors in killing leukemic progenitors from BCR/ABL positive acute leukemia. Leuk. Res. 2010, 34, 1035-1042.
15. Hayes, F. Toxins-antitoxins: plasmid maintenance, programmed cell death, and cell cycle arrest. Science 2003, 301, 1496-1499.
16. Sevin, E. W.; Barloy-Hubler, F. RASTA-Bacteria: a web-based tool for identifying toxin-antitoxin loci in prokaryotes. Genome Biol. 2007, 8, R155.
17. Yamaguchi, Y.; Inouye, M. Regulation of growth and death in Escherichia coli by toxin-antitoxin systems. Nat Rev Microbiol 2011, 9, 779-790.
18. Unterholzner, S. J.; Poppenberger, B.; Rozhon, W. Toxin-antitoxin systems. Bioengineered 2014, 5, 1-13.
19. Brantl, S. Bacterial type I toxin-antitoxin systems. RNA Biol. 2012, 9, 1488-90.
20. Fozo, E. M.; Hemm, M. R.; Storz, G. Small toxic proteins and the antisense RNAs that repress them. Microbiol Mol Biol Rev 2008, 72, 579-89, Table of Contents.
21. Pedersen, K.; Gerdes, K. Multiple hok genes on the chromosome of Escherichia coli. Mol. Microbiol. 1999, 32, 1090-1102.
22. Gerdes, K.; Bech, F. W.; Jørgensen, S. T.; Løbner-Olesen, A.; Rasmussen, P. B.; Atlung, T.; Boe, L.; Karlstrom, O.; Molin, S.; von Meyenburg, K. Mechanism of postsegregational killing by the hok gene product of the parB system of plasmid R1 and its homology with the relF gene product of the E. coli relB operon. EMBO J. 1986, 5, 2023-9.
23. Gerdes, K.; Rasmussen, P. B.; Molin, S. Unique type of plasmid maintenance function: postsegregational killing of plasmid-free cells. Proc. Natl. Acad. Sci. U. S. A. 1986, 83, 3116-3120.
24. Kawano, M.; Oshima, T.; Kasai, H.; Mori, H. Molecular characterization of long direct repeat (LDR) sequences expressing a stable mRNA encoding for a 35-amino-acid cell-killing peptide and a cis-encoded small antisense RNA in Escherichia coli. Mol. Microbiol. 2002, 45, 333-349.
25. Kawano, M. Divergently overlapping cis-encoded antisense RNA regulating toxin-antitoxin systems from E. coli: hok/sok, Idr/rdl, symE/symR. RNA Biol. 2012, 9, 1520-7.
26. Wiewrodt, R.; Amin, K.; Kiefer, M.; Jovanovic, V. P.; Kapoor, V.; Force, S.; Chang, M.; Lanuti, M.; Black, M. E.; Kaiser, L. R.; Albelda, S. M. Adenovirus-mediated gene transfer of enhanced Herpes simplex virus thymidine kinase mutants improves prodrug-mediated tumor cell killing. Cancer Gene Ther. 2003, 10, 353-364.
27. Wang, Q.; Li, W.; Liu, X. S.; Carroll, J. S.; Jänne, O. A.; Krasnickas, E.; Chinnaiyan, A. M.; Pienta, K. J.; Brown, M. NIH Public Access. 2014, 27, 380-392.
28. Yang, W. S.; Park, S.-O.; Yoon, A.-R.; Yoo, J. Y.; Kim, M. K.; Yun, C.-O.; Kim, C.-W. Suicide cancer gene therapy using pore-forming toxin, streptolysin O. Mol. Cancer Ther. 2006, 5, 1610-1619.
29. Prados, J.; Melguizo, C.; Rama, A. R.; Ortiz, R.; Segura, A.; Boulaiz, H.; Vélez, C.; Caba, O.; Ramos, J. L.; Aránega, A. Gef gene therapy enhances the therapeutic efficacy of doxorubicin to combat growth of MCF-7 breast cancer cells. Cancer Chemother. Pharmacol. 2010, 66, 69-78.
30. Ortiz, R.; Prados, J.; Melguizo, C.; Rama, A. R.; Ávarez, P. J.; Rodriguez-Serrano, F.; Caba, O.; Boulaiz, H.; Aranega, A. Gef gene therapy enhances the therapeutic efficacy of cytotoxics in colon cancer cells. Biomed. Pharmacother. 2012, 66, 563-567.
31. Gribbon, P. Morphology and ATP-based Cytotoxicity Profiling of Cancer Cell Lines using the EnSight Multimode Plate Reader with Well Imaging Technology.
32. Garbow, N. Cell Counting and Confluency Analysis as Quality Controls in Cell-Based Assays.
33. Tang, Y.; Jackson, M.; Qian, K.; Phillips, M. I. Ischemia Gene Therapy. 2002, 695-699.
34. Dalle, B.; Payen, E.; Beuzard, Y. Modulation of transduced erythropoietin expression by iron. Exp. Hematol. 2000, 28, 760-764.
35. Coulter, J. a; McCarthy, H. O.; Worthington, J.; Robson, T.; Scott, S.; Hirst, D. G. The radiation-inducible pE9 promoter driving inducible nitric oxide synthase radiosensitizes hypoxic tumour cells to radiation. Gene Ther. 2008, 15, 495-503.
36. Gossen, M.; Bujard, H. Tight control of gene expression in mammalian cells by tetracycline-responsive promoters. Proc. Natl. Acad. Sci. U. S. A. 1992, 89, 5547-51.
37. Yao, F.; Theopold, C.; Hoeller, D.; Bleiziffer, O.; Lu, Z. Highly Efficient Regulation of Gene Expression by Tetracycline in a Replication-Defective Herpes Simplex Viral Vector. Mol. Ther. 2006, 13, 1133-1141.
38. Loew, R.; Heinz, N.; Hampf, M.; Bujard, H.; Gossen, M. Improved Tet-responsive promoters with minimized background expression. BMC Biotechnol. 2010, 10, 81.
39. Vanderveen, N.; Paran, C.; Krasinkiewicz, J.; Zhao, L.; Palmer, D.; Hervey-Jumper, S.; Ng, P.; Lowenstein, P.; Castro, M. Effectiveness and preclinical safety profile of doxycycline to be used "off-label" to induce therapeutic transgene expression in a Phase I clinical trial for glioma. Hum. Gene Ther. Clin. Dev. 2013, 126, 116-126.
40. Giménez, E.; Lavado, A.; Giraldo, P.; Cozar, P.; Jeffery, G.; Montoliu, L. A transgenic mouse model with inducible Tyrosinase gene expression using the tetracycline (Tet-on) system allows regulated rescue of abnormal chiasmatic projections found in albinism. Pigment Cell Res. 2004, 17, 363-370.
41. Wang, Z. X.; Bian, H. B.; Yang, J. S.; De, W.; Ji, X. H. Adenovirus-mediated suicide gene therapy under the control of Cox-2 promoter for colorectal cancer. Cancer Biol. Ther. 2009, 8.
42. Rama, A. R.; Aguilera, A.; Melguizo, C.; Caba, O.; Prados, J. Tissue Specific Promoters in Colorectal Cancer. Dis. Markers 2015, 2015.
43. Shieh, G. S.; Shiau, A. L.; Yo, Y. Te; Lin, P. R.; Chang, C. C.; Tzai, T. S.; Wu, C. L. Low-dose etoposide enhances telomerase-dependent adenovirus-mediated cytosine deaminase gene therapy through augmentation of adenoviral infection and transgene expression in a syngeneic bladder tumor model. Cancer Res. 2006, 66, 9957-9966.
44. Deharvengt, S.; Rejiba, S.; Wack, S.; Aprahamian, M.; Hajri, A. Efficient electrogene therapy for pancreatic adeno carcinoma treatment using the bacterial purine nucleoside phosphorylase suicide gene with fludarabine. Int. J. Oncol. 2007, 30, 1397-1406.

## Claims

**1.** An isolated RNA or DNA polynucleotide having an identity of at least
a) 80%,
b) 85%,
c) 90%
d) 95%
e) 99%
with nucleotide sequence SEQ ID NO: 1, for use as a medicinal product.

**2.** The isolated RNA or DNA polynucleotide for use according to the preceding claim, with nucleotide sequence SEQ ID NO: 1.

**3.** A genetic structure comprising at least one of the following types of sequences:
a) a nucleotide sequence comprising at least one polynucleotide according to any of claims 1-2 for *in vitro* or *in vivo* transcription,
b) a nucleotide sequence corresponding to a gene expression system or vector comprising a polynucleotide according to any of claims 1-2, operatively linked with at least one promotor which directs the transcription of said nucleotide sequence, and with other sequences necessary or suitable for transcription and the suitable regulation thereof in time and place, for use in medicine, or alternatively, for use as a medicinal product.

**4.** A cell comprising a polynucleotide according to any of claims 1-2, or a genetic structure according to claim 3.

**5.** A composition comprising:
a) a polynucleotide according to any of claims 1-2,
b) a genetic structure according to claim 3, and/or
c) a cell according to claim 4,
for use as a medicinal product.

**6.** The polynucleotide according to any of claims 1-2, the genetic structure according to claim 3, the cell according to claim 4, and/or the composition according to claim 5, for the prevention, improvement, relief, or treatment of a proliferative disease.

**7.** The polynucleotide, the genetic structure, the cell, and/or the composition for use according to claim 6, wherein the proliferative disease is cancer.

**8.** The polynucleotide, the genetic structure, the cell, and/or the composition for use according to claim 6, wherein the proliferative disease is breast cancer.

**9.** The polynucleotide, the genetic structure, the cell, and/or the composition for use according to claim 6, wherein the proliferative disease is colon cancer.

**10.** The polynucleotide, the genetic structure, the cell, and/or the composition for use according to claim 6, wherein the proliferative disease is cervical cancer.

**11.** A gene construct comprising at least one of the following types of sequences:
a) a nucleotide sequence comprising the polynucleotide according to any of claims 1-2, and an isolated RNA or DNA polynucleotide having an identity of at least
I) 80%,
II) 85%,
III) 90%
IV) 95%
V) 99%
with nucleotide sequence SEQ ID NO: 2,
for *in vitro* or *in vivo* transcription,
b) a nucleotide sequence corresponding to a gene expression system or vector comprising the polynucleotide according to any of claims 1-2, and the polynucleotide as described in a), operatively linked with at least one promotor which directs the transcription of said nucleotide sequence, and with other sequences necessary or suitable for transcription and the suitable regulation thereof in time and place.

**12.** A combined preparation comprising:
a) the polynucleotide according to any of claims 1-2, and
b) the polynucleotide as described in claim 11 a).

**13.** The genetic structure according to claim 11, or the combined preparation according to claim 12, for use as a medicinal product.

**14.** The combined preparation according to claim 12 for simultaneous or sequential separately combined administration for the prevention, improvement, relief, or treatment of a proliferative disease.

**15.** The combined preparation for use according to claim 12, wherein the proliferative disease is cancer.

**16.** The combined preparation for use according to claim 12, wherein the proliferative disease is breast cancer.

**17.** The combined preparation for use according to claim 12, wherein the proliferative disease is colon cancer.

**17.** The combined preparation for use according to claim 12, wherein the proliferative disease is cervical cancer.
